# EUROPEAN PATENT SPECIFICATION

(11) **EP 2 476 745 B1**
(45) Date of publication and mention of the grant of the patent: **01.04.2015**
(21) Application number: 11151003.8
(22) Date of filing: 14.01.2011
(51) Int. Cl.: G01N 33/14

(54) **Cultivation plate system and method for the improved detection of microorganisms which contaminate food products**
Kultivierungsplattensystem und Verfahren zur verbesserten Erkennung von Mikroorganismen, die Lebensmittelprodukte kontaminieren
Système de plaque de culture pour une détection améliorée de micro-organismes qui contaminent des produits alimentaires

(43) Date of publication of application: 18.07.2012
(73) Proprietor: BioSilta Oy, 90014 Oulu (FI)
(72) Inventor: Vasala, Antti, 90500 Oulu (FI)
(74) Representative: Ganahl, Bernhard

(56) References cited:
- US-A- 4 906 573
- SHANK J L ET AL: "Agar medium to define acid producing bacteria", 1 May 1957 (1957-05-01), JOURNAL OF BACTERIOLOGY, AMERICAN SOCIETY FOR MICROBIOLOGY, WASHINGTON, DC; US, PAGE(S) 625 - 626, XP009149372, ISSN: 0021-9193 * the whole document *
- TASKILA SANNA ET AL: "Comparison of Enrichment Media for Routine Detection of Beer Spoiling Lactic Acid Bacteria and Development of Trouble-shooting Medium for Lactobacillus backi", 1 January 2010 (2010-01-01), JOURNAL OF THE INSTITUTE OF BREWING, INSTITUTE OF BREWING. LONDON, GB, PAGE(S) 151 - 156, XP009149374, ISSN: 0046-9750 * abstract * * page 154, column 2, paragraph 2 *
- SHAMTSYAN M ET AL: "Lactic acid batch and fed-batch fermentation directly from starch-containing substrates", 1 March 2004 (2004-03-01), JOURNAL OF BIOLOGICAL PHYSICS AND CHEMISTRY, ASSOCIATION OF MODERN SCIENTIFIC INVESTIGATION, GE, PAGE(S) 17 - 21, XP009149375, ISSN: 1512-0856 * abstract *

## Description

The present invention relates to the enrichment and detection of microorganisms which contaminate food products, especially beverages and soft-drinks. It is particularly suitable for the detection of beer-spoiling bacteria. The present invention provides a novel system which a) improves the recovery of spoilage microbes b) provides faster detection and earlier emergence of colonies on plates, and c) provides larger colony sizes which makes detection easier.

Since bacteria can propagate in and contaminate food products after the product has passed the normal quality control at the end of the production process and is already packed, bottled or canned, spoilage by microorganisms (mostly anaerobes) can cause significant economical losses. Suspicious material needs to be removed from the market until its un-spoilt nature can be confirmed. In the worst case the costs for recollecting the spoiled food from food stores can be significant. Furthermore, the delivery of poor-quality products to customers can easily have disadvantages to the reputation of the producer and the trademark. Just one bacterium in a food container or bottle can cause spoilage of the product. Therefore the occurrence of even a low number of bacterial cells in a package should be detected. This applies particularly to beverages like beer. In general, beer is a hostile environment for other microbes than yeasts due to the presence of antibacterial hop compounds and ethanol, anaerobic conditions and a relatively low pH. However, some microorganisms can still proliferate in beer and deteriorate the product by causing turbidity, acidity or by the production of an unfavorable smell (e.g. hydrogen sulfide).

Beer-spoilers are mostly identified by methods based on the incubation in culture media. The incubation medium can be liquid or solid (e.g. agar-plates). According to a survey conducted by Sartorius AG (Goettingen, Germany) liquid nutrient cultures are usually better than solid culture media because microbial invasion is immediately visible by signs of fermentation. Especially slightly damaged organisms are more able to recuperate and proliferate in liquid cultures than on solid culture plates. However, exact microbial detection in liquid cultures takes a considerably longer time (typically 5 to 10 days) compared to plate cultures where results can be often obtained after 2 to 3 days. For the microbiological control, also many advanced biotechnological techniques, such as immunoassay and polymerase chain reaction (PCR), have been applied. However, even for these "quick methods", several days of cultivation may be necessary to reach an amount of bacteria sufficient for a noticeable signal. Nevertheless, plate-cultivation methods have maintained their important role in microbial quality control system in the food producing industry and breweries for the following reason: 1) they are easy and inexpensive to use, 2) quantification of the contaminant microbes is possible, 3) several contaminant microbes can be detected at the same time simultaneously, 4) further characterization and identification of the contaminant microbe is easy since "pure isolates" can be obtained directly from plate in the form of bacterial colonies. In breweries samples are typically taken from fermentation tanks, storage tanks, over-pressure tanks and surfaces. Many of these locations are anaerobic, but a contamination source can also exist on aerobic surfaces. Some strict anaerobic species can also survive in an oxygen-containing environment if they are protected by bio-film or isolated from oxygen (for example occurring in liquid droplets). Consequently, surfaces in aerobic environments can serve as contamination sources for anaerobic microbes. Many of the (anaerobic) contaminants are fairly slow growing. In normal quality assurance systems in breweries they are detected by plating technology. Since even a single bacterial cell can cause product contaminations, samples are often concentrated from large volumes by filtration. In practice, beer samples are filtrated through a membrane filter which retains the microorganisms present in the sample. The membrane is then removed from the filter and transferred onto a cultivation plate. Only in very few places the possibility exists to perform sample treatment under completely anaerobic conditions. If, however, the conditions are not anaerobic the spoilage bacteria are subjected to a severe oxidative stress which may kill or damage them. This means that they either do not form colonies on plates or their revitalization takes longer. A damaged cell when suddenly exposed to nutrient-rich conditions may die.

Anaerobes do not tolerate oxygen because they utilize metabolic routes built around enzymes that react with oxidants. They also miss the key components present in aerobic organisms which can detoxify oxygen: namely catalase and oxidase enzyme complexes. Therefore, with these organisms it is important to ensure that the time when they are exposed to harmful oxygen is minimized. Also their metabolic activity during the exposure to oxygen should be kept minimal. Unfortunately, normal sampling and the setup of plate cultivation occur in oxygen-containing conditions, and the generation of anaerobic conditions in anaerobe jars can take several hours. It should also be noted that even after the removal of oxygen from the environment, fast activation of damaged cells due to high nutrient environment (especially high sugar content of the cultivation plates) may further damage or kill them. These problems are generally difficult to overcome because in order to facilitate fast growth and early detection (appearance of colonies), the culture medium should provide a high amount of nutrients.

Oxygen-sensitive organisms are typically cultivated in anaerobic incubators, anaerobe chambers or anaerobe pouches. Such products are available from several manufacturers like Oxoid, BD and Merck. The chemicals in such anaerobic systems typically generate hydrogen gas which reacts quickly with oxygen by generating water. Hydrogen can be derived, for example, from the reaction between sodium borohydride and water. The reaction, for example, between citric acid and sodium bicarbonate can generate both CO₂ and hydrogen. Different variants of such systems have been launched but none of them can immediately provide anaerobic conditions. Systems providing fully anaerobic conditions for sample treatment and cultivation have been available for several years but they have been applied only in few breweries. For example, the Modular Atmosphere Controlled System (MACS) from Oxoid can guarantee an optimal atmosphere for anaerobic organisms. In optimal cases all the steps (sampling, sample treatment like filtration, plating) are to be performed under anaerobic conditions. Such devices are rarely available for standard quality analysis units of the food industry.

Even those microbes which, in principle, prefer oxygenic conditions may suffer from oxidative stress when exposed to suddenly changing glucose concentrations. High glucose concentrations can induce metabolic (fermentative) pathways that result in the production of growth-inhibiting compounds like ethanol, acetic acid and lactic acid.

Typically food-spoilage bacteria are cultivated on medium plates which contain an enzymatic digest of proteins, meat extract, salts (like ammonium citrate, Mg-sulfate or chloride, phosphates as potassium or sodium salts) and fairly high concentration of sugars (typically 20 g/L of glucose). If the cultivation of beer-spoiling bacteria is intended beer is normally included into these medium compositions since the presence of beer and its hop compounds can prevent the growth of organisms that cannot propagate in beer. In some cultivation media other sugars than glucose (e.g. fructose) or other carbon sources (like lactates) can be applied. Many of these cultivation media also contain acetate (as potassium or sodium salts) and phosphates, which serve as buffering components. Generally used cultivation media for beer-spoiling lactic acid bacteria include MRS Lactobacilli broth supplemented with 25 % beer, Raka Ray, and NBB-medium (Nachweismedium für Bierschädliche Bakterien).

Generally, to facilitate the early detection of contaminants, cultivation plate systems usually try to maximize growth and colony size. Therefore high amounts of glucose or other sugars are normally applied. However, as mentioned above fast activation of the metabolism may cause further damages or even the death of stressed cells. Therefore, a slow glucose release system was developed to feed gradually a reasonably high amount of glucose to bacteria. In this regard some products applicable to simple shaken liquid cultivations have recently emerged e.g. the feed-bead system (Jeude et al., Biotechnol. Bioeng. 95(3):433-445, 2006) and enzymatic glucose delivery system of BioSilta Oy (Panula-Perälä et al., Microb. Cell Fact. 7: 31, 2008). Taskila et al. (J. Inst. Brew. 116(2), 151-156, 2010) have recently shown that a slow glucose release can provide faster enrichment of *Lactobacillus backi* and *Pediococcus damnosus* in liquid cultures. This system was based on the enzymatic release of glucose from starch released from a starch-agar layer to a liquid medium. In practice the beer-spoiling organisms were growing in MRS-based liquid medium that was set on the top of a starch-agar gel. However, so far none of these slow-release systems have been adapted to plate cultivation.

Despite the fact that in many anaerobic systems the accumulation of CO₂ may render the cultivation environment acidic (and additionally many microbes make acidic metabolites), relatively little attention has been paid to the pH optimization of plate cultures. Usually the pH of plate media have been set to levels which have been optimized for liquid cultivations. This has disadvantages, since unlike in liquid media the mentioned acidic metabolites on plates cannot disperse freely but will accumulate within a restricted area around the microbial colony due to the limited diffusion possibilities. Accumulation of these metabolites may restrict the growth of bacteria and limit the colony size. In addition, it is known that the occurring short chain fatty acids, like acetic acid, propionic acid or lactic acid, are antibacterial in high doses. Their inhibitory effect can be, however, diminished by converting into their salts. For the cultivation of lactic acid bacteria in liquid cultures, CaCO₃ is sometimes used to neutralize the harmful acids and thereby increases the cell yield. CaCO₃ is sometimes also included in solid media, for example in yeast cultivation plates. But so far very little attention has been paid to the development of CaCO₃-plates having a consistent quality and desired pH. Direct mixing of a CaCO₃ slurry with nutrient agar results in the fast sedimentation of CaCO₃ onto the bottom of the plates. Thus, the efficient concentration of CaCO₃ on the top of the plate may be fairly low. In the method developed by Wade et al. (J. Bacteriol. 51 (6):787-788, 1946) CaCO₃ was added by a pipette into each cultivation plate which was then cooled quickly to minimize the sedimentation of CaCO₃ onto the bottom of the plate. This system was further improved by Shank and Silliker (J. Bacteriol. 73 (5): 625-626, 1956) by introducing CaCO₃ in a colloidal form (melted mixture of carrageenan ("irish moss"), agar and CaCO₃) which could be distributed onto the plate more evenly. In this publication of Shank et al. (1956), CaCO₃ was primarily used to retard the diffusion rate of acids, thus allowing the improved detection of the acid-producing colonies by pH-indicator dyes, like chromo-cresol blue. In neither of these systems, the pH-elevating property of CaCO₃ was considered to set a certain pH for the plates. CaCO₃ itself is a slight alkaline and it raises the pH level of medium plates. Since CaCO₃ is easily decomposed in acidic conditions during heat treatment, the pH-level of the CaCO₃-slurry cannot be directly adjusted.

Thus, it the object of the present invention to provide a cultivation plate system and a method for the fast enrichment and detection of microorganisms which contaminate food products, especially beverages and soft-drinks. These cultivation plates shall avoid the above described disadvantages of the known plate cultivation systems and shall be suitable to grow and detect food-contaminating microorganisms quickly and reliably.

This object is solved by the subject matter of claims 1 and 10. Preferred embodiments are the subject matter of the dependent claims.

In particular, the present invention provides a cultivation plate system for the detection of food product-contaminating microorganisms selected from the group consisting of Lactobacillus, Pediococcus, Pectinatus, Megasphaera, Enterobacterium, Bacillus or Campylobacter, comprising as separate entities:
(a) a cultivation plate having a pH of 5.7 to 7.0 containing a nutrient base, 5-40 g/l di-, oligo- or polysaccharide, 5-30 g/l CaC03 in a colloidal form and a gelling agent
(b) a stock solution of an enzyme in a dilution containing 10-100 units/ml,
wherein the enzyme digests a di-, oligo- or polysaccharide to glucose.

>

According to the present invention a "food product" is any nutrient product taken up by humans or animals for nutrition purposes. It may be a solid, jelly or liquid. In a particular preferred embodiment it is a beverage, like beer or a soft-drink.

According to the present invention the "microorganisms to be detected" are those undergoing stress when exposed to oxygen. These microorganisms are preferably bacteria, in particular aerobes, facultative or strict anaerobes or microaerophilic. The most prominent groups of beer-spoiling organisms are lactobacilli (e.g. *L. brevis, L. lindneri, L. backi*), pediococci (*P. damnosus, P. pentosaceus*), *Pectinatus* (e.g. *P. frisingensis, P. cerevisiiphilus*) and *Megasphaera.* Among the important food poisoning microbes, enterobacteria (like *Salmonella*) and bacilli are facultative whereas campylobacteria microaerophilic and clostridia are strict anaerobes. Although facultative organisms, like Salmonella, are mostly cultivated under aerobic conditions since they grow much faster with oxygen, slow nutrient release as provided with the system of the present invention can help the damaged cells to survive from oxidative stress.

The cultivation plate can be based on any appropriate nutrient medium composition as nutrient base. These media and their ingredients are commercially available or applied as described in the literature and well known to a person skilled in the art. These culture media are either known as complex media which are composed of at least partly less well defined raw materials, like extracts (e.g. yeast extract, meat extract) or hydrolysates (e.g. peptone, casamino acids), or as defined media which are mixtures of chemicals with known composition.

These media may contain inorganic elements, mineral salts (e.g. calcium, potassium, natrium, magnesium, mangan, sulfate, bicarbonate, chloride) and/or ammonia source(s) (ammonia, nitrate, amino acids). Additionally they may optionally contain vitamins (e.g. ascorbic acid, riboflavin, folic acid, thiamin, vitamin A), amino acids (e.g. L-cysteine, glycine), growth factors (e.g. EGF, IGF, TGF), antibiotics (streptomycin, tetracycyline, chloramphenicol), cytokins, serum proteins (e.g. BSA, HSA), ionic or non-ionic tensides (e.g. polysorbates, Tween^{R}80) and/or nucleosides (e.g. ribonucleosides, desoxyribonucleosides). If the detection of beer-spoiling bacteria is intended the medium should contain a suitable amount (e.g. 20-30 %, preferably 25%) of beer.

Suitable standard media for the growth of microorganisms are for example Peptone-Yeast Extract Broth, Staphylococcus Broth, PPLO Media, Mannitol Salt Broth, Luria-Bertani Broth, DMEM, RPMI, BME, Fischer's medium or Trypticase Soy Broth. Preferably, MRS (Mann-Rogosa-Sharpe) lactobacilli medium composition is used.

As a complex carbon source the plate media contain at least a di-, oligo- or polysaccharide that serves as a precursor or raw material for enzymatic glucose production. Preferably starch or starch derivatives are used in an amount of 5 to 40 g/l, more preferably 10 to 20 g/l. It is particularly preferred to use potato starch in an amount of 10 to 20 g/l. To facilitate the desired slow release of a carbon source as a growth-limiting substrate, the plate medium composition should contain, if at all, only a small amount (preferably less than 1 g/L) of a monosaccharide, e.g. glucose. However, most preferred is that the plate medium does not contain any monosaccharide, in particular glucose, as a carbon source.

In addition, the plate media contain a gelling agent. Examples for gelling agents are agar, agarose, alginate, carrageenans, cellulose and combinations thereof. A particular preferred combination is agar and carrageenan.

A further ingredient of the plate media is colloidal CaCO₃ in an amount of 5 to 30 g per liter, preferable 30 g per liter.

The pH of the plate media may be adjusted to 5.7 to 7.0 with a suitable acid (e.g. HCl). pH values lower than 5.7 would cause partial degradation of the agar during heat treatment and should be therefore avoided. For the ready to use plates the pH is preferably >6, most preferred between 6.2 and 6.8.

A particular preferred system comprises a starch-containing nutrient medium solidified with agar and containing CaCO₃ which is added in a colloidal form (i.e. as a mixture of carrageenan and CaCO₃) that can be evenly distributed into the medium. In this regard it has been found advantageous to adjust the above mentioned desired pH level before casting the plates by the addition of about 15-30% HCl, preferably about 20 % HCl, after combining the cultivation medium (e.g. MRS/starch/agar) with a CaCO₃/carrageenan slurry.

The second component of the new cultivation plate system is a stock solution of a di-, oligo- or polysaccharide digesting enzyme. Preferred is a glucoamylase stock solution, preferably in a dilution containing 10-100 units/ml, most preferably about 20 units/ml. "1 unit" is defined as an amount needed to split one micromole of maltose per minute at 30°C at pH 4.8. According to the present invention a small drop of the polysaccharide splitting enzyme (preferably glucoamylase) is added on the surface of the cultivation plate before applying the bacterial sample onto the plate. Preferably 1 unit of enzyme is delivered in one 50 µl drop. This approach provides a slow enzymatic substrate delivery system which can gradually deliver a carbon source (e.g. glucose) for growing bacteria.

The bacterial sample can be applied onto the plate by several methods. Bacteria may be collected from a large volume by filtration, which after the bacteria-containing membrane is placed over the enzyme drop. Alternative, samples can be collected from surfaces by a moist cotton-swab, which is used to spread bacteria onto plate and simultaneously spread the enzyme drop. After applying the contaminant sample, the cultivation plate is transferred into aerobic incubator (chamber, jar, pouch) and cultivated at appropriate temperature (typically 27 to 30 °C) until colonies appear on the plate.

It is within the general knowledge of a person skilled in art how to prepare cultivation plates for the detection of microorganisms. In this regard reference is made to general laboratory books like "Molecular Cloning: A Laboratory Manual" by J. Sambrook and D. Russell. In a preferred embodiment of the present invention the plates are prepared as follows:
(A) Colloidal CaCO₃ is prepared by heating carrageenan and anhydrous CaCO₃ (e.g. in a microwave oven) in a suitable amount of water. After the carrageenan has melted, the mixture is kept for at least 1 hour at about 80°C and mixed with a magnetic stirrer. Thereafter the mixture is sterilized (e.g. by autoclaving for 15 min at 121 °C). The mixture is kept > 80 °C until combined with medium base (B)
(B) Medium base (B) is a suitable cultivation medium for the microorganism to be detected. A person skilled in the art knows which microorganism grows best in which medium. For the detection of lactobacilli and pediococci modified MRS (Man-Rogosa-Sharpe) lactobacillus broth may be used. In a preferred embodiment it is prepared by dissolving the following components in a suitable amount of water: proteose peptone, meat extract, yeast extract, glucose, Tween^{R}80 (polysorbate 80), ammonium citrate, sodium acetate, magnesium sulfate * 6H₂O, dipotassium phosphate. Also chemicals (e.g. L-cysteine or MnSO4) known to promote the survival of anaerobic bacteria can be further added. If the detection of beer-spoiling bacteria is intended the medium should contain a suitable amount (e.g. 25 %) of beer. The pH of the medium base may be adjusted to 5.7 to 7.0 with a suitable acid (e.g. HCl). pH values lower than 5.7 would cause partial degradation of the agar during heat treatment and should be therefore avoided. Thereafter a suitable amount of starch and agar is added. Then the medium is sterilized (e.g. autoclaved for 15 min. at 121 °C) and kept at about 80 °C until gel casting. At this point, a suitable amount of a mineral acid (e.g. HCl) can be added to set a desired pH for the plates (c.f. Example 3, Table 1) To prepare the final composition, (A) and (B) are combined by mixing, in particular in a proportion of 1 part (A) to 4 parts (B). Then the final composition is poured into Petri dishes and solidified.
(C) Before the microorganism sample is given onto the plates, a starch-splitting enzyme solution is given on the surface of the plates. After applying the sample, the cultivation plate is transferred into an incubator and cultivated at appropriate temperature (typically 27 to 30 °C) until colonies appear on the plate.

The invention utilizes the slow enzymatic substrate delivery system which can gradually deliver a carbon source (e.g. glucose) for growing bacteria. Gradual glucose delivery is preferred since the early and fast activation of the metabolism of oxygen-sensitive (anaerobic or microaerophilic) organisms may lead to lethal injuries in their cultivation conditions where some oxygen is still left. Gradual glucose delivery is also beneficial for oxygen-tolerating bacteria because quick changes in the glucose concentration can induce oxidative stress (decrease the viability) and/or lead to accumulation of growth-inhibiting metabolites. This issue is particularly important since the contamination organisms may already have reduced viability due to the stressful growth conditions and the stress caused by sample treatment. In addition, the invention applies an improved system for the neutralization of the growth-inhibiting metabolites.

The new cultivation plate system provides faster emergence of colonies, improved survival rate and provides larger colony size by some or all of the following mechanisms: 1) slower activation of metabolism due to slow glucose release, 2) decreased accumulation of harmful metabolites 3) neutralization of short chain fatty acids like acetic acid, lactic acid and propionic acid due to a high effective concentration of CaCO₃, 4) the presence of CaCO₃ may provide higher efficient amount of CO₂ (and consequently reduced O₂ concentration), 5) enhanced pH-level maintenance is obtained by buffering with colloidal CaCO₃.

It has been found out in the present invention that for a fast and easy detection of contaminants plate cultures are preferred over liquid cultures. The additional advantages of plate cultures are 1) colony-forming microbes can be fast evaluated with respect to size, opacity or visibility, and morphology, and may be microscopically identified 2) several different contaminant species can be detected and isolated on a single plate. Unlike many modern molecular methods plate cultivation methods do not require extensive expertise or expensive devices.

The invention is described in further detail with regard to the accompanying figures:

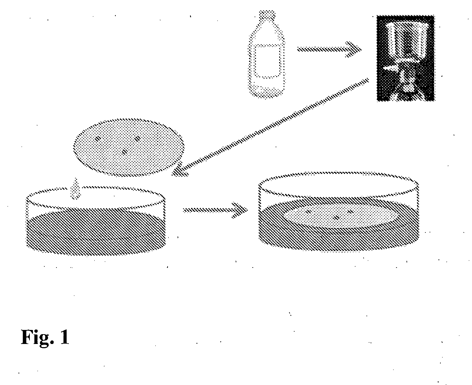

Fig. 1: Use of the cultivation plates for determining spoilage bacteria concentrated from beer. Bacteria are concentrated from a beer sample onto a nitrocelluose membrane. Before placing the membrane onto cultivation, a drop of glucoamylase enzyme is added.

The invention is further described with respect to the following examples.

### Example 1: Preparation and use of the enrichment plates

This example shows how MRS-medium supplemented with beer was improved with the technology of the present invention. Colloidial CaCO₃ (referred hereafter as (A)) was added to a cultivation medium (hereafter referred as (B)). In this example in total 1000 ml were prepared by combining 200 ml of (A) as a hot solution with 800 ml of (B).

### Preparation of the plates

(A) Colloidal CaCO₃ was prepared by heating 4.5 g carrageenan and 30 g anhydrous CaCO₃ in a microwave oven in 200 ml water. After the carrageenan had melted, the mixture was kept at least 1 hour in a hot water bath (about 80°C) and mixed with a magnetic stirrer. Thereafter the mixture was sterilized by autoclaving 15 min at 121 °C. The mixture was kept > 80 °C until combined with (B). If solidified it could be melted in microwave oven prior to combining with hot (B).
(B) Medium base (B) is a 1.25-folded concentrate of a selected cultivation medium. In this example modified MRS (Man-Rogosa-Sharpe) lactobacillus broth was used. It was prepared by dissolving the following components resulting in a final volume of 800 ml: proteose peptone (10 g), meat extract (10 g), yeast extract (5 g), glucose (1 g), Tween^{R}80 (polysorbate 80) (1 g), ammonium citrate (2 g), sodium acetate (5 g), magnesium sulfate * 6H₂O (0.1 g), dipotassium phosphate (2 g), beer 250 ml and water 550 ml. The pH of the medium was adjusted to 5.7 with 37 % HCl. Thereafter 20 g of soluble potato starch and 15 g agar were added. The medium was autoclaved 15 at 121 °C and kept at 80 °C until gel casting. To prepare the final composition, 200 ml of (A) and 800 ml of (B) were combined by mixing. Each 20 ml aliquot of the final composition was poured into Petri dishes. If the plates were intended to be used for samples possibly containing yeast cells, they were supplemented with the antibiotics cycloheximide (preferred dosing is 1 µl/litre). Without any further pH adjustment, the pH-level of the plates would be typically 6.9 ±0.1 due to the pH-elevating nature of CaCO₃. To obtain plates having pH 6.2, 3 ml of 18.5 % HCl was added to the medium base (B) before it was combined to colloidal CaCO₃ (A).

### Use of the plates for the cultivation

In this example soluble starch immobilized in agar gel works as the major glucose source for the bacteria. Starch cannot be directly used as the carbon source by most of the beerspoilage bacteria. However, it can be used as a supplier for glucose. Therefore 1 unit, i.e. a drop (about 50 µl), from a glucoamylase enzyme stock solution (15 units/ml) is added onto the plate. Glucoamylase penetrates into the gel matrix where it gradually releases glucose from starch. The bacteria-containing sample can either directly wiped onto the plate or the bacteria can be first concentrated onto a membrane filter (see Fig. 1) which is placed over the enzyme drop.

Example 1 shows how the CaCO₃-based neutralization system and enzyme-based glucose-delivery system can be applied with medium compositions which are suitable for the cultivation of beer-spoiling bacteria, especially lactobacilli and pediococci.

### Example 2:

### Optimization of the enzyme dosage needed to provide a sufficient amount of glucose

The sizes of the bacterial colonies are more or less dependent on the amount of sugar in the medium of the plates. 20 g/L glucose (an amount which is present in many cultivation media) was generally found to provide the largest colony size. Thus, in the present invention it was desired to obtain this amount of glucose, not from the beginning on but within 1 to 2 days of cultivation. The determination of the amount of glucoamylase needed for this purpose was performed by using MRS-agar plates (Petri- plates having 85 mm diameter) supplemented with 20 g/L starch but no CaCO₃ colloid. Enzyme amounts of 0.2 -0.5 - 1 - 5 - 10 - 20 units were spread onto a ~ 17 cm² surface area. The appropriate amount of enzyme was defined as an amount which provides clear zones (i.e. areas with degraded starch) within 24 hours.

It was found that a concentration of 1 unit provided within a 50 µl drop is a sufficient amount of enzyme for the current technology. With a higher enzyme dosage starch was degraded much earlier (within few hours), with a lower enzyme dosage the amount of released glucose from starch was estimated to be insufficient for large colony sizes. Thus, the availability of glucose was considered to be dependent on the enzyme dosage. This result provides a possibility to tune the plate system for achieving different glucose release rates.

### Example 3:

### Applying CaCO₃ into the plate medium composition and adjusting the desired pH level of the plates

The method developed by Shank and Silliker (J. Bacteriol. (1956) Vol 73: 625-626) was found to provide CaCO₃-containing plates with consistent structure. In that method a slurry prepared from melted carrageenan ("Irish moss") and CaCO₃ was combined with a hot agar-containing medium composition prior to plate casting. In the present invention this system was further modified so that 1.5-fold higher CaCO3 and carrageenan concentrations were applied. The CaCO₃/carrageenan slurry raised the pH level when combined with the cultivation medium composition (MRS/starch/agar/beer). Therefore, methods to adjust the pH level of the plates were tested.

The pH level of the CaCO₃/carrageenan slurry could not be directly adjusted since the addition of HCl induced a fast decomposition of CaCO₃ to CO₂. The alternative method, setting the pH of the MRS/starch/agar to a low level (even below pH 4) and then combining with the CaCO₃ was not successful either, although the desired pH level (pH 6) could be reached. The quality of the resulting gels became very poor since the structure of the gelling components (agar and carrageenan) is destroyed during the autoclavation under acidic conditions (i.e. at a pH much lower than 6).

The following system for pH adjustment has been developed by the present inventors. The desired pH level of about pH 6.0 - 6.8 was adjusted by the addition of 18.5 % HCl after combining the cultivation medium (MRS/starch/agar) with the CaCO3/carrageenan slurry. The correspondence between the amount of acid added and the resulting pH of the plates is shown in Table 1. The pH level of the plates was tested by allowing 5 ml of water to lie on the plate surface with occasional shaking and subsequent measuring of the pH of the water phase. The plate batches for pH 6.2 and 6.8 were also tested for long-term properties. These plates were found to maintain their pH properties well for several days. This indicates that the gel structure of these plates can stabilize the CaCO₃ fairly well and diminish the reactions (e.g. release of CO₂) between the slightly acidic medium and CaCO₃.

**Table 1. Correspondence between the volume of 18.5 % HCl and the pH level of the plates. Prior to the addition of the CaCO₃/carrageenan mixture (providing 5 g CaCO₃ and 0.9 g carrageenan to the final composition), pH of the medium had been set to 5.7**

| **ml of 18.5 % HCl added** | **pH after 1 hour** | **pH after 4 hours** |
|---|---|---|
| 6 ml | 6.03 | 6.13 |
| 3 ml | 6.27 | 6.29 |
| 1.5 ml | 6.53 | 6.54 |
| 0.75 ml | 6.60 | 6.65 |

### Example 4:

In examples 4 and 5, the following strains obtained from VTT culture collection were used: *Pediococcus damnosus* E-97848, *Lactobacillus brevis* E-89347, and *Lactobacillus backi* E-052886.

### Determination of colony sizes

During the performed cultivations the plates of the present invention provided larger colony sizes than the control plates having the same amount of glucose (20 g/L) available but without containing any CaCO₃. This phenomenon was obviously attributed to the acid-neutralizing properties of the plate. Due to the presence of colloidial CaCO₃ non-used plates are quite turbid. However, during the cultivation process large bacterial colonies were often surrounded by clear zones, indicating that the produced acids (lactic acid) have reacted with calcium carbonate by generating Ca-salts (e.g. Ca-lactate) which have a much better solubility compared to CaCO₃. This assumption could be confirmed by manually applying lactic acid onto the plates which generated also clear areas on the plate. In the present innovation it has been shown that the inventive system can enhance the growth of food-spoiling bacteria by efficiently neutralizing the acids produced by bacteria. Lactic acid bacteria can be sensitive to a pH decrease caused by the accumulation of lactic acid which they produce. Solid CaCO₃ has been successfully used to maintain their fermentative activities and growth in liquid cultivation media (Hong et al. 1996 "Growth of Lactobacillus acidophilus in whey-based medium and preparation of cell concentrate for production of probiotics. J. Microbiol. Biotechnol. 6 (2):128-131).

**Table 2. Colony size comparison between MRS/beer plates and plates of Example 1. The same medium components (MRS-medium and beer) were used to in both medium compositions. Prior to plating, bacteria were stressed 40 h in beer.**

| **Organism/Plate** | **Colony diameter** | | | | |
|---|---|---|---|---|---|
| | Days after plating | | | | |
| | 2 | 3 | 4 | 5 | 6 |

| *Pediococcus damnosus* | | | | | |
|---|---|---|---|---|---|
| MRS/Beer agar | - | 0.5-1 mm | 0.5-1 mm | | |
| Enz/starch/CaCO₃ | 0.5 mm | 0.5-3 mm | 0.5-3 mm | | |

| *Lactobacillus backi* | | | | | |
|---|---|---|---|---|---|
| MRS/Beer agar | - | - | - | - | 0.1 mm |
| Enz/starch/CaCO₃ | - | - | 0.2 mm | 0.5 mm | 0.7-1.5 mm |

| *Lactobacillus brevis* | | | | | |
|---|---|---|---|---|---|
| MRS/beer agar | - | 0.2 mm | 0.2 mm | 0.5 mm | |
| Enz/starch/CaCO₃ | | 0.2 mm | 0.5 mm | 1 mm | |

### Example 5:

### Faster detection of Pediococcus and Lactobacillus on plates

Pure cultures of three lactic acid bacteria species, *Pediococcus damnosus* and *Lactobacillus backi* and *Lactobacillus brevis*, were prepared. These bacteria were first cultivated in MRS Lactobacillus broth (Merck) at 30°C, harvested by centrifugation (3500 rpm for 3 hours) and then exposed to beer (lager, 4.5 % EtOH content) for 40 hours (30°C). Defined amounts (400 or 2000 bacterial cells estimated on the basis of the culture turbidity) of bacteria were distributed into 25 ml of saline solution (0.9 % NaCl), and the bacteria were harvested by vacuum filtration onto nitrocellulose membranes. The membrane was placed onto a plate according to Example 1 after addition of a drop of glucoamylase (15 U/ml). As a control, MRS/beer agar-plates medium (which contains the normal amount (20 g/L) glucose) was used. As shown in Table 3, for *Pediococcus* cultivations first colonies emerged after 3 or 4 days, but the survivability in the plates of the present invention was much higher compared to the control plates (normal MRS-medium supplemented with beer). With Anaerocult A (Merck AG, Darmstadt, Germany) reagents, pediococci refused to grow in such control plates. This suggests that also the generation of CO₂ in sufficient amounts may be important for the survival and some microaerophilic bacteria. The present innovation however seems to provide good results for both Anaerocult A and Anaerocult C systems. In *Lactobacillus backi* cultivations, colonies appeared after 4 and 6 days, respectively. With all the tested bacteria, the survivability was very much improved by the use of the new plate products.

**Table 3. Strains tested and the number of days before the first colonies were observed using Anaerocult A. The beer-spoiling organisms were stressed 40 hours in beer (4.5 % lager) at 30 °C prior to plating, and collected onto NC-membrane (0.25 um pore size) which was placed onto the plate. Plates were cultivated in an anaerobic chamber with Anaerocult A reagents (for P. damnosus also with Anaerocult C reagents).**

| **Strain** | **Enz/starch/CaCO₃** | | **MRS/beer control** | |
|---|---|---|---|---|
| | Colonies visible | % Survivability | Colonies visible | % Survivability |
| *Lactobacillus brevis* | Day 3 | 7-12% | Day ¾ | 3-7% |
| *Lactobacillus backi* | Day 4 | 1.3-2.5% | Day 6 | 0.4-1.25% |
| *Pediococcus damnosus* | Day 3 or 4 | 1.5% | Not detected | 0% |
| Using Anaerocult C | | | | |
| *Pediococcus damnosus* | Day 3 | 0.5 - 1.5% | Day 3 | 0.1-0.25% |

Anaerocult A (Merck) is used for cultivation of strictly anaerobic microbes. According to the studies of Imhof and Heinzer 1996 ("Continuous monitoring of oxygen concentrations in several systems for cultivaton of anaerobic bacteriaJ. Clinical Microbiol 34 (7):1646-1648) it provides < 0.5 % oxygen concentrations within 60 to 93 minutes.

Anaerocult C (Merck) is used for the specific incubation of microaerophilic and capneic (CO₂-needing) microorganisms. Gas concentrations produced are CO₂ 8-10 %, 0₂ 5-6 %.

## Claims

1. A cultivation plate system for the detection of food product-contaminating microorganisms selected from the group consisting of Lactobacillus, Pediococcus, Pectinatus, Megasphaera, Enterobacterium, Bacillus or Campylobacter, comprising as separate entities:
(a) a cultivation plate having a pH of 5.7 to 7.0 containing a nutrient base, 5-40 g/l di-, oligo- or polysaccharide, 5-30 g/l CaCO₃ in a colloidal form and a gelling agent
(b) a stock solution of an enzyme in a dilution containing 10-100 units/ml, wherein the enzyme digests a di-, oligo- or polysaccharide to glucose.

2. The cultivation plate system of claim 1, wherein the food product is a beverage.

3. The cultivation plate system of claim 2, wherein the beverage is beer.

4. The cultivation plate system of claim 3, wherein the cultivation plate (a) additionally contains beer.

5. The cultivation plate system of any of claims 1 to 4, wherein the gelling agent is agar and/or carrageenan.

6. The cultivation plate system of any of claims 1 to 5, wherein the polysaccharide is starch or a starch derivative.

7. The cultivation plate system of any of claims claim 1 to 6, wherein the enzyme of component (b) is glucoamylase.

8. The cultivation plate system of claim 7, wherein the concentration of glucoamylase in the stock solution is 20 U/ml.

9. The cultivation plate system of any of claims 1-8, wherein the nutrient medium is Man-Rogosa-Sharpe (MRS) lactobacillus broth.

10. A method for the detection of food products-contaminating microorganisms selected from the group consisting of Lactobacillus, Pediococcus, Pectinatus, Megasphaera, Enterobacterium, Bacillus or Campylobacter, comprising the following steps:
(A)preparing a sterile colloidal CaCO₃ solution having a concentration of 5-30 g/l CaCO₃
(B) preparing a sterile nutrient medium containing nutrients, 5-40 g/l di-, oligo- or polysaccharide and at least a gelling agent wherein the pH of the nutrient medium is adjusted in the range of 5.0 to 7.0,
(C) mixing (A) and (B),
(D) adjusting the pH to 5.7 to 7.0,
(E) pouring the resulting composition into Petri dishes,
(F) adding a di-, oligo- or polysaccharide splitting enzyme solution from a stock solution containing 10-100 units/ml of said enzyme onto the surface of the nutrient gel in the plate,
(G) applying a microorganism sample and transferring the cultivation plate into an incubator and incubating the plate at an appropriate temperature until colonies appear on the plate.

11. The method of claim 10, wherein the colloidal CaCO₃ solution contains carrageenan.

12. The method of claim 10 or 11, wherein the polysaccharide is starch or a starch derivative.

13. The method of any of claims 10 to 12, wherein the enzyme is glucoamylase.

## Patentansprüche

1. Kultivierungsplattensystem für die Detektion von Nahrungsmittelprodukt-kontaminierenden Mikroorganismen, ausgewählt aus der Gruppe bestehend aus Lactobacillus, Pediococcus, Pectinatus, Megasphaera, Enterobacterium, Bacillus oder Campylobacter, umfassend als separate Einheiten:
(a) eine Kultivierungsplatte, welche einen pH von 5,7 bis 7,0 aufweist und eine Nahrungsgrundlage enthält, 5-40 g/l, Di-, Oligo- oder Polysaccharid, 5-30 g/l CaCO₃ in einer kolloidalen Form und ein Gelierungsmittel
(b) eine Stocklösung eines Enzyms in einer Verdünnung enthaltend 10-100 Einheiten/ml, wobei das Enzym ein Di-, Oligo- oder Polysaccharid zu Glucose verdaut.

2. Das Kultivierungsplattensystem von Anspruch 1, wobei das Nahrungsmittelprodukt ein Getränk ist.

3. Das Kultivierungsplattensystem von Anspruch 2, wobei das Getränk Bier ist.

4. Das Kultivierungsplattensystem von Anspruch 3, wobei die Kultivierungsplatte (a) zusätzlich Bier enthält.

5. Das Kultivierungsplattensystem nach einem der Ansprüche 1 bis 4, wobei das Gelierungsmittel Agar und/oder Carrageenan ist.

6. Das Kultivierungsplattensystem nach einem der Ansprüche 1 bis 5, wobei das Polysaccharid Stärke oder ein Stärkederivat ist.

7. Das Kultivierungsplattensystem nach einem der Ansprüche 1 bis 6, wobei das Enzym der Komponente (b) Glucoamylase ist.

8. Das Kultivierungsplattensystem nach Anspruch 7, wobei die Konzentration von Glucoamylase in der Stocklösung 20 U/ml ist.

9. Das Kultivierungsplattensystem nach einem der Ansprüche 1 bis 8, wobei das Nährmedium Man-Rogosa-Sharpe (MRS) Lactobacillus-Brühe ist.

10. Ein Verfahren für die Detektion von Nahrungsmittelprodukten-kontaminierenden Mikroorganismen, ausgewählt aus der Gruppe bestehend aus Lactobacillus, Pediococcus, Pectinatus, Megasphaera, Enterobacterium, Bacillus oder Campylobacter, umfassend die folgenden Schritte:
(A) Zubereiten einer sterilen kolloidalen CaCO₃-Lösung, welche eine Konzentration von 5-30 g/l CaCO₃ hat.
(B) Zubereiten eines sterilen Nährmediums enthaltend Nährstoffe, 5-40 g/l Di-, Oligo- oder Polysaccharid und mindestens ein Gelierungsmittel, wobei der pH des Nährmediums auf dem Bereich von 5,0 bis 7,0 angepasst wird,
(C) Mischen von (A) und (B),
(D) Anpassen des pH auf 5,7 bis 7,0,
(E) Gießen der resultierenden Zusammensetzung in Petrischalen,
(F) Zugeben einer Di-, Oligo- oder Polysaccharid spaltenden Enzymlösung aus einer Stocklösung enthaltend 10-100 Einheiten/ml des Enzyms auf die Oberfläche des Nährgels in der Platte,
(G) Auftragen einer Mikroorganismus-Probe und Transferieren der Kultivierungsplatte in einen Inkubator und Inkubieren der Platte bei einer geeigneten Temperatur bis Kolonien auf der Platte erscheinen.

11. Das Verfahren nach Anspruch 10, wobei die kolloidale CaCO₃-Lösung Carrageenan enthält.

12. Das Verfahren nach Anspruch 10 oder 11, wobei das Polysaccharid Stärke oder ein Stärkederivat ist.

13. Das Verfahren nach einem der Ansprüche 10 bis 12, wobei das Enzym Glucoamylase ist.

## Revendications

1. Système de plaque de culture pour la détection de micro-organismes qui contaminent des produits alimentaires choisis parmi le groupe composé de Lactobacillus, Pediococcus, Pectinatus, Megasphaera, entérobactérie, Bacillus ou Campylobacter, comprenant comme entités séparées:
(a) une plaque de culture ayant un pH de 5,7 à 7,0 contenant une base de substances nutritives, de 5 à 40 g/l de disaccharide, oligosaccharide ou polysaccharide, de 5 à 30 g/l de CaCO₃ sous forme colloïdale et un agent gélifiant,
(b) une solution mère d'une enzyme dans une dilution contenant de 10 à 100 unités/ml, où l'enzyme digère un disaccharide, oligosaccharide ou polysaccharide, pour obtenir du glucose.

2. Système de plaque de culture selon la revendication 1, dans lequel le produit alimentaire est une boisson.

3. Système de plaque de culture selon la revendication 2, dans lequel la boisson est de la bière.

4. Système de plaque de culture selon la revendication 3, dans lequel la plaque de culture (a) contient en outre de la bière.

5. Système de plaque de culture selon l'une quelconque des revendications 1 à 4, dans lequel l'agent de gélification est l'agar et/ou le carraghénane.

6. Système de plaque de culture selon l'une quelconque des revendications 1 à 5, dans lequel le polysaccharide est de l'amidon ou un dérivé d'amidon.

7. Système de plaque de culture selon l'une quelconque des revendications 1 à 6, dans lequel l'enzyme du composant (b) est une glucoamylase.

8. Système de plaque de culture selon la revendication 7, dans lequel la concentration de glucoamylase dans la solution mère est de 20 U/ml.

9. Système de plaque de culture selon l'une quelconque des revendications 1 à 8, dans lequel le milieu de substances nutritives est un bouillon de lactobacillus de Man-Rogosa-Sharpe (MRS).

10. Procédé pour la détection de micro-organismes qui contaminent des produits alimentaires choisis parmi le groupe composé de Lactobacillus, Pediococcus, Pectinatus, Megasphaera, entérobactérie, Bacillus ou Campylobacter, comprenant les étapes suivantes consistant à:
(A) préparer une solution de CaCO₃ colloïdal stérile ayant une concentration de 5 à 30 g/l de CaCO₃
(B) préparer un milieu de substances nutritives stérile contenant des substances nutritives, de 5 à 40 g/l de disaccharide, oligosaccharide ou polysaccharide et au moins un agent gélifiant, où le pH du milieu de substances nutritives est ajusté dans la plage de 5,0 à 7,0,
(C) mélanger (A) et (B),
(D) ajuster le pH à de 5,7 à 7,0,
(E) verser la composition résultante dans des boîtes de Petri,
(F) ajouter une solution d'enzyme de division de disaccharide, oligosaccharide ou polysaccharide d'une solution mère contenant de 10 à 100 unités/ml de ladite enzyme sur la surface du gel nutritif dans la plaque,
(G) appliquer un échantillon de micro-organisme et transférer la plaque de culture dans un incubateur et incuber la plaque à une température appropriée jusqu'à ce que des colonies apparaissent sur la plaque.

11. Procédé selon la revendication 10, dans lequel la solution de CaCO₃ colloïdal contient du carraghénane.

12. Procédé selon la revendication 10 ou 11, dans lequel le polysaccharide est de l'amidon ou un dérivé d'amidon.

13. Procédé selon l'une quelconque des revendications 10 à 12, dans lequel l'enzyme est une glucoamylase.
